# EUROPEAN PATENT APPLICATION

(11) **EP 4 012 039 A1**
(43) Date of publication of application: **15.06.2022**
(21) Application number: 20213866.5
(22) Date of filing: 14.12.2020
(51) Int. Cl.: C12P 7/10, C12M 1/00

(54) **METHOD AND SYSTEM OF INCREASING THE FERMENTABILITY OF MICROBIAL FERMENTATION**

(71) Applicant: Sekab E-Technology AB, 891 26 Örnsköldsvik (SE)
(72) Inventor: SIBBESEN, Ole, 2880 Bagsvaerd (DK); CAVKA, Adnan, 891 96 Arnäsvall (SE)
(74) Representative: Kransell & Wennborg KB

(57) **Abstract**

The present disclosure relates to a method of increasing the fermentability of at least a part of a microbial fermentation process involving co-fermentation of sugars from lignocellulosic biomass to fermentation products. The method comprises the steps of:
- providing a first lignocellulosic biomass-based stream and a second lignocellulosic biomass-based stream different to the first lignocellulosic biomass-based stream, the first and second lignocellulosic biomass-based streams together comprising xylose, glucose and mannose;
- mixing the first and second lignocellulosic biomass-based streams to reach a ratio of xylose to mannose within a target range;
- treating the mixed first and second lignocellulosic biomass-based streams, or treating the first and second lignocellulosic biomass-based streams separately prior to the step of mixing, to provide a fermentation media comprising xylose, glucose and mannose, wherein the mixing of the first and second lignocellulosic biomass-based streams results in a ratio of xylose to mannose in the fermentation media within a target range;
- co-fermenting the fermentation media by means of fermentation microorganisms of yeast to a fermentation product.

## Description

### TECHNICAL FIELD

The present disclosure generally relates to a method and system of increasing the fermentability of a microbial fermentation process involving co-fermentation of sugars from lignocellulosic biomass to fermentation products. The invention also relates to the use of mannose to increase the fermentation rate of a microbial fermentation process involving co-fermentation of a fermentation media comprising at least glucose and xylose.

### BACKGROUND

Lignocellulosic residues from forestry are attractive as feedstocks for the production of green chemicals and fuels, since they are abundant, relatively inexpensive, and not used for food. Lignocellulose consists mainly of lignin and two classes of polysaccharides, cellulose and hemicellulose. Cellulose is composed of polysaccharide chains of several hundred to over ten thousand linked glucose units, whereas hemicellulose is a polysaccharide composed of xylose, other pentose sugars and various hexose sugars, e.g. glucose and mannose. In lignocellulose, cellulose and hemicellulose are tightly associated to lignin, a polyphenolic compound that ties the cellulose and hemicellulose polymers together, thus providing the lignocellulose with rigidity and mechanical strength.

For example, in the production of ethanol from lignocellulosic materials, various pretreatment and hydrolysis steps are typically used to separate cellulose, hemicellulose and lignin and subsequently to degrade the cellulose and hemicellulose polysaccharides to fermentable saccharides in a fermentation media, often referred to as "hydrolysate". The fermentable saccharides are then converted to ethanol by means of fermenting microorganisms, such as yeast or bacteria, and the ethanol is recovered by means of distillation. The yeast Saccharomyces cerevisiae, for example, metabolizes hexose sugars and is a microorganism suitable for industrial processes for cellulosic bioethanol production. However, Saccharomyces cerevisiae does not naturally ferment xylose that comprises a major part of many types of lignocellulosic material, why much attention has been put forth to engineering yeast to also acquire the ability to ferment xylose to ethanol and therefore to more fully utilize the available sugars from lignocellulose.

However, even with current engineered yeast being able to ferment xylose, the fermenting rate of xylose is significantly slower than that of glucose. The fermentation rate of xylose has been found to correlate to the glucose concentration. It seems that the xylose is taken up by the same transmembrane transporters as glucose, but that the transmembrane transporters have a lower affinity towards xylose than towards glucose. Additionally, synthesis of the transmembrane transporters is induced by glucose but not by xylose, meaning that the amount of transmembrane transporters gets much lower when glucose has been exhausted and xylose is the only remaining fermentable sugar.

While the improvements related to xylose fermentation have led to an increased yield of ethanol based on sugars from lignocellulose, there are still potential improvements to be realized related to the ability and rate of xylose fermentation.

### SUMMARY

In view of the above, it is an object of the present invention to provide improvements with respect to methods and arrangements for fermentation of lignocellulosic biomass, particularly to achieve a more favourable process by facilitating the simultaneous fermentation, or co-fermentation, of mannose and xylose.

According to a first aspect of the invention, a method of increasing the fermentability of at least a part of a microbial fermentation process involving co-fermentation of sugars from lignocellulosic biomass to fermentation products is provided. The method comprises the steps of:
- providing a first lignocellulosic biomass-based stream and a second lignocellulosic biomass-based stream different to the first lignocellulosic biomass-based stream, the first and second lignocellulosic biomass-based streams together comprising xylose, glucose and mannose;
- mixing the first and second lignocellulosic biomass-based streams;
- treating the mixed first and second lignocellulosic biomass-based streams, or treating the first and second lignocellulosic biomass-based streams separately prior to the step of mixing, to provide a fermentation media comprising xylose, glucose and mannose, wherein the mixing of the first and second lignocellulosic biomass-based streams results in a ratio of xylose to mannose in the fermentation media within a target range;
- co-fermenting the fermentation media by means of fermentation microorganisms of yeast to a fermentation product.

Thus, two separate lignocellulosic biomass-based streams are mixed to reach a target ratio of xylose to mannose in the fermentation media. The inventors have realized that the fermentation, and fermentation rate, of xylose may be increased by co-fermentation with mannose, and in particular for a specific relative amount of mannose to xylose. Hereby, the overall fermentation time can be reduced. Moreover, the inventors have realized that such beneficial co-fermentation of mannose and xylose occurs in particular when the co-fermentation in addition to mannose and xylose, also include glucose. Co-fermenting of the fermentation media is referring to the co-fermentation of fermentable sugars in the fermentation media, e.g. glucose, mannose and xylose.

The mixing of the first and second lignocellulosic biomass-based streams may be performed prior to pretreating the lignocellulosic biomass, i.e. subjecting the lignocellulosic biomass to a pretreatment process. The pretreatment process is typically carried out in a pretreatment arrangement, comprising an impregnation vessel and a reactor vessel, or alternatively a combined impregnation and reactor vessel. During the impregnation, the lignocellulosic biomass is impregnated with an additive facilitating degradation of the lignocellulosic biomass, and in the reactor the degradation is improved by exposing the lignocellulosic biomass to an elevated temperature and pressure. The pretreatment arrangement also generally comprises an inlet for receiving the lignocellulosic biomass, e.g. the mixed first and second lignocellulosic biomass-based streams, to be pretreated and an outlet for discharging the pretreated lignocellulosic biomass, typically as a biomass slurry.

The mixing of the first and second lignocellulosic biomass-based streams may alternatively be performed subsequently to the pretreatment process, and e.g. prior a hydrolysis step of the pretreated lignocellulosic biomass. During hydrolysis of the pretreated lignocellulosic biomass, the pretreated lignocellulosic biomass is subject to enzymatic hydrolysis by means of saccharification enzymes. Thus, each of the first and second lignocellulosic biomass-based streams may be a first and second pretreated biomass slurry which have been separately pretreated, e.g. by different pretreatment processes considering any differences between the first and second lignocellulosic biomass-based streams, where-after they are mixed to form a mixed biomass slurry, which are to be subjected to hydrolysis.

The mixing of the first and second lignocellulosic biomass-based streams may alternatively be performed subsequent to the hydrolysis step, but prior to any fermentation step. Thus, each of the first and second lignocellulosic biomass-based streams may be a first and second hydrolysate which have been separately pretreated and hydrolysed, e.g. by different pretreatment processes and hydrolysing considering any differences between the first and second lignocellulosic biomass-based streams, where-after they are mixed to form a fermentation media which are to be subjected to fermentation.

Regardless of when, or where in the process of from providing lignocellulosic biomass raw material, to prior to fermentation, the mixing of the first and second lignocellulosic biomass-based streams is carried out, it is performed to reach a ratio of the xylose to mannose within a target range in the fermentation media. For example, if the mixing of the first and second lignocellulosic biomass-based streams is performed prior to the pretreatment process, the yield of mannose and xylose during the process up to the step of fermentation need to be considered in order to reach the target range of the ratio of xylose to mannose in the fermentation media. If on the other hand, mixing of the first and second lignocellulosic biomass-based streams is performed just prior to the step of fermentation (i.e. subsequent to the step of hydrolysis), any known or measured content of xylose and mannose in the first and second lignocellulosic biomass-based streams may be considered to reach the target range of the ratio of xylose to mannose in the fermentation media.

According to at least one example embodiment, the mixing of the first and second lignocellulosic biomass-based streams are performed prior to the step of co-fermenting.

It should be noted that the target range of the ratio of xylose to mannose in the fermentation media may be reached by various measures, e.g. diluting a high mannose or xylose containing lignocellulosic biomass-based stream and mixing it with a lignocellulosic biomass-based stream only containing mannose or xylose in the appropriate amount, or simply combining two lignocellulosic biomass-based streams for which the xylose and mannose content are suitable for reaching the target range of the ratio of xylose to mannose in the fermentation media. For example, the target range of the ratio of xylose to mannose in the fermentation media may be reached by intentionally adding mannose or a mannose comprising substance (e.g. being the second lignocellulosic biomass-based stream) to a lignocellulosic biomass-based stream comprising xylose and glucose (e.g. being the first lignocellulosic biomass-based stream), to provide a mannose containing lignocellulosic biomass material to be further processed to provide said fermentation media. That is, co-fermenting the fermentation media in the presence of an intentionally increased mannose concentration, as compared to a mannose concentration which would be present if only the first lignocellulosic biomass-based stream had been processed without the intentional addition of mannose, results in an improved co-fermentation of xylose and mannose.

According to at least one example embodiment, increase or decrease of the xylose, and/or increase or decrease of the mannose, as compared to first or second lignocellulosic biomass-based streams, may be used to reach the target range of the ratio of xylose to mannose in the fermentation media. For example, the mixing of the first and second lignocellulosic biomass-based streams may result in that the ratio of xylose to mannose is changed by at least 5 % as compared to a ratio of xylose to mannose of either the first or the second lignocellulosic biomass-based streams. Alternatively, the mixing of the first and second lignocellulosic biomass-based streams may result in that the concentration of mannose and/or the concentration of xylose is changed (decreased or increased) by at least 5% in relation to the corresponding concentrations in the the first or the second lignocellulosic biomass-based streams. For example, by mixing hardwood (relatively high xylose and relatively low mannose) with softwood (relatively high mannose and relatively low xylose) in an appropriate amount, the target range of the ratio of xylose to mannose in the fermentation media may be reached.

According to at least one example embodiment, the target range is different to a ratio of xylose to mannose in the separate first and/or second lignocellulosic biomass-based streams.

Hereby, the target range of the ratio of xylose to mannose in the fermentation media can be reached by mixing the first and/or second lignocellulosic biomass-based streams, while this is not possible by processing and fermenting the first and/or second lignocellulosic biomass-based streams separately.

According to at least one example embodiment, the target range is a predefined target range, the predefined target range being based on an increased fermentation rate during at least a part of the co-fermentation of the fermentation media.

Thus, the specific relative amount of mannose to xylose in the fermentation media, resulting in that the fermentation, or fermentation rate, of xylose is increased during at least a part of the co-fermentation, may be predefined, or determined in advance. That is, by knowing the preferred relative amounts of xylose and mannose in relation to the fermentation rate, and defining such preferred relative amounts as a predefined target range of the ratio of xylose to mannose, the desired improvement in the fermentation may be achieved.

According to at least one example embodiment, the part of the co-fermentation of the fermentation media for which the predefined target range causes an increased fermentation rate comprises the fermentation phase where the utilisation of fermentable sugar changes from primarily glucose to primarily xylose.

Thus, the part is related to the fermentation phase in which a change in fermentation substrate, or a change in the fermentation tendency, from glucose to xylose occurs. Thus, during at least a part of the fermentation process from the start of fermentation to a point in which all fermentable sugars of glucose, mannose and xylose have been fermented, the part being a part in which all glucose, or almost all glucose, has been fermented (i.e. glucose is exhausted) and the yeast changes to mainly or primarily ferment xylose, or co-ferment (e.g. simultaneously ferment) mannose and xylose. Such part may be referred to as a fermentation changeover from glucose to xylose, or from hexoses to pentoses. Stated differently, the fermentation process may be divided into different phases, such as the glucose fermentation phase and the xylose fermentation phase, wherein the fermentation changeover is a transition phase from the glucose fermentation phase to the xylose fermentation phase.

According to at least one example embodiment, the target range is extending from 0.08 to 13.

Such target range is advantageous with regards to improved fermentation. The ratio of xylose to mannose may e.g. be based on the concentrations of xylose and mannose, or on the respective amount of xylose and mannose to the total amount of fermentable sugars in the fermentation media, and/or to the amount of xylose, mannose and glucose.

According to at least one example embodiment, the target range is extending from 0.08, or from 0.1, or from 0.2 or from 0.3 or from 0.5 or from 0.7 or from 0.9, or from 1, or from 1.5, or from 1.7, or from 2, or from 3. Additionally, or alternatively the target range is at most 12, or 11, or 10, or 8, or 7, or 5. According to at least one example embodiment, the target range is extending from 0.5 to 12, or from 1 to 10, or from 1.5 to 8, or from 2 to 7.

According to at least one example embodiment, the target range is extending from 1.2 to 5, such as e.g. from 2 to 5, e.g. 2.5 to 5. For example, the target range may extend from 1.2 to 2.5, or from 1.2 to 2. According to at least one example embodiment, an alternative target range based on the ratio of xylose to mannose and glucose, i.e. xylose to the hexose, in which hexose corresponds to the sum of mannose and glucose. For example, the alternative target rage may extend from 0.33 to 1.5, such as e.g. from 0.45 to 1.5, e.g. 0.6 to 1.5. For example, the alternative target range may extend from 0.33 to 0.6, or from 0.33 to 0.45.

According to at least one example embodiment, the amount of xylose in the fermentation media is between 5 and 65 wt%, and the amount of mannose in the fermentation media is between 5 and 65 wt%, as compared to the total amount of glucose, mannose and xylose.

Such amounts of xylose and mannose are advantageous with regards to improved fermentation. According to at least one example embodiment, the amount of xylose in the fermentation media is between 10 and 60 wt%, or between 15 and 55 wt%, or between 20 and 50 wt%, such as e.g. between 25 and 45 wt%, and the amount of mannose in the fermentation media is between 10 and 60 wt%, or between 15 and 55 wt%, or between 20 and 50 wt%, such as e.g. between 25 and 45 wt%, as compared to the total amount of glucose, mannose and xylose. According to at least one example embodiment, the amount of xylose in the fermentation media is between 25 and 60 wt%, or between 31 and 60 wt%, or between 37.5 and 60 wt%, or between 25 and 37.5 wt% as compared to the total amount of glucose, mannose and xylose, and the amount of mannose in the fermentation media is between 6 and 31 wt%, or between 7.5 and 31 wt%, or between 12.5 and 31 wt%, such as e.g. between 15 and 31 wt%, or between 6 and 20 wt%, as compared to the total amount of glucose, mannose and xylose.

According to at least one example embodiment, the amount of glucose in the fermentation media is between 5 and 65 %, such as between 10 and 60 wt%, or between 15 and 55 wt%, or between 20 and 50 wt%, such as e.g. between 25 and 45 wt% as compared to the total amount of glucose, mannose and xylose. According to at least one example embodiment, the amount of glucose in the fermentation media is between 20 and 62.5 %, such as between 31 and 62.5 wt%, or between 47.5 and 62.5 wt%, or between 20 and 55 wt%, such as e.g. between 20 and 47.5 wt% as compared to the total amount of glucose, mannose and xylose. According to at least one example embodiment, the total amount of xylose, mannose and glucose amounts to 100 %, wherein the amount of xylose and mannose are defined as previously described, and the amount of glucose in the fermentation media sums up to 100 %.

The target range, and/or the amount of xylose in the fermentation media between 5 and 65 wt%, and/or the amount of mannose in the fermentation media between 5 and 65 wt% and any amount of glucose between 5 and 65 wt%, may be referring to an initial values (i.e. initial target range, initial amount of xylose, initial amount of mannose and initial amount of glucose), i.e. the value prior to the start of fermentation (typically for a batch fermentation) or may be referring to the set-point value (i.e. the set point target range, set point amount of xylose, set point amount of mannose and set point glucose), i.e. the set value, or steady state value, of the fermentation (typically for a continuous fermentation).

According to at least one example embodiment, the fermentation media comprises xylose, mannose and glucose as the only three fermentable sugars in the fermentation media. However, according to at least one example embodiment, the fermentation media comprises galactose and/or arabinose. For example, the fermentation media may comprise 15-20 g/L of xylose, 40-45 g/L of mannose, 25-30 g/L of glucose, 8-10 g/L of galactose, and 1-2 g/L of arabinose.

According to at least one example embodiment, the step of co-fermenting is performed in a continuous, or semi-continuous, manner in a fermentation vessel.

Continuous, or semi-continuous fermentation, may be preferred over batch-wise fermentation, as the production of the fermentation product may be increased and/or simplified. The fermentation vessel may thus be arranged and configured for continuous fermentation. However, according to at least one example embodiment, the step of co-fermenting is performed in a batchwise manner in a fermentation vessel. The fermentation vessel may thus be arranged and configured for batch-wise fermentation. In a batchwise fermentation process, the fermentation media is fed to a vessel in which the sugars from the lignocellulosic biomass is fermented into fermentation products by means of fermentation microorganisms of yeast.

According to at least one example embodiment, the method comprises the steps of:
- measuring of a residual sugar indicator parameter RSI, which parameter directly or indirectly indicates the concentration of residual sugars, during fermentation in the fermentation vessel;
- determining an RSI setpoint based on a rate of change of the measured residual sugar indicator parameter, such that the RSI setpoint corresponds to a maximum rate of change; and
- automatically adapting the amount of fermentable sugars of glucose, mannose and xylose in the fermentation media in response to the measured residual sugar indicator parameter RSI and the RSI setpoint, so as to achieve and maintain the RSI setpoint, whereby efficient co-fermentation of sugars to fermentation products is obtained.

For example, the adaptation of the amount of fermentable sugars of glucose, mannose and xylose in the fermentation media may be achieved by adapting the first and/or the second lignocellulosic biomass-based streams. By knowing the ratio of xylose to mannose such that the target range of the xylose to mannose ratio is reached in the fermentation media as previously described, and adapting the amount of fermentable sugars of glucose, mannose and xylose in the fermentation media so that the RSI setpoint is reached and/or kept, an improved fermentation rate is achieved. The maximum rate of change typically relates to a setpoint in which the fermentation rate of the co-fermentation of the fermentation media is the highest. According to at least one example embodiment, the setpoint corresponds to a maximum fermentation rate (or consumption rate) of the sugars in the fermentation media, i.e. a maximum overall fermentation rate. According to at least one example embodiment, the setpoint corresponds to a fermentation phase in which the glucose has been exhausted, or a fermentation phase in which the glucose and/or mannose concentration is low enough (e.g. lower than 5 g/L) so the consumption rate of glucose and/or mannose is below that of xylose and mannose (or any hexose). The method may further comprise the step of providing an initial active population of the fermentation microorganisms of yeast into the fermentation vessel. Typically, the RSI setpoint is adapted to be within the changeover phase as previously described, or is adapted to be within the fermentation phase in which xylose is being co-fermented (simultaneously) with the mannose (or any hexose).

According to at least one example embodiment, the step of automatically adapting the amount of fermentable sugars of glucose, mannose and xylose comprises automatically adapting the ratio of xylose to mannose in a predetermined manner. Thus, the ratio of xylose to mannose may be adapted in a predetermined manner such that the target range of the xylose to mannose ratio is reached in the fermentation media as previously described, in response to the measured residual sugar indicator parameter RSI and the RSI setpoint.

According to at least one example embodiment, the RSI setpoint corresponds to a steady state condition in the fermentation vessel and the method further comprises in situ growing of the fermentation microorganisms in the fermentation vessel by means of the treated and mixed first and second lignocellulosic biomass-based streams to the fermentation vessel, so as to maintain a steady-state population of fermentation microorganisms in the fermentation vessel, whereby further addition of microorganisms is not required. The RSI setpoint may, according to one embodiment, comprises a target interval of the RSI or a target interval of the RSI rate of change, and the step of automatically adapting in turn comprises the steps of: comparing measured, and preferably processed, values of the residual sugar indicator parameter to the RSI setpoint; and adjusting the amount of treated and mixed first and second lignocellulosic biomass-based streams to the fermentation vessel in response to the comparison, so as to reach and stay within a narrow range around the RSI setpoint, preferably corresponding to 20 % of glucose, mannose and xylose, or corresponding to +/- 10 g/sugar per liter, or more preferably +/- 5 g sugar per liter, or most preferably +/- 2 g sugar per liter, e.g. measured as a time average over a predetermined time.

According to at least one example embodiment the residual sugar indicator parameter RSI is measured directly on the fermentation media, e.g. measured online.

According to at least one example embodiment, the method comprises the step of intentionally increasing the ethanol concentration of the fermentation media, and performing the step of co-fermenting the fermentation media in the presence of an intentionally increased ethanol concentration.

That is, the ethanol concentration is intentionally increased (e.g. with at least 2 wt%) compared to not intentionally increasing the ethanol concentration. The intentionally increased ethanol concentration (e.g. of at least 2 wt%) may be referred to as an elevated ethanol concentration, or intentionally elevated ethanol concentration. The ethanol concentration may be increased compared to not intentionally increasing the ethanol concentration, of at least 4 wt%, or at least 6 wt% or at least 8 wt%, or at least 10 wt%.

Hereby, the xylose fermentation time can be reduced. The inventors have realized that an increased or elevated ethanol concentration promotes the fermentation rate for a fermentation media comprising xylose, at least during the part of the fermentation process where xylose is fermented. It is believed that the increased or elevated ethanol concentration equalizes the individual fermentation rates of the various sugars comprised in the fermentation media, and that the mannose and xylose are fermented with a more equal fermentation rate compared to a co-fermentation of the same fermentation media in the presence of an ethanol concentration which is not intentionally increased. In other words, the relative fermentation rate of xylose to the fermentation rate of mannose is increased. The intentionally increased ethanol concentration may be achieved by adding ethanol, or an ethanol producing substance.

According to at least one example embodiment, the term "intentionally elevated ethanol concentration" is simply referred to as an "elevated ethanol concentration". The intentionally elevated ethanol concentration is referring to a the ethanol concentration, which at least during a portion of the fermentation process, that is higher than the ethanol concentration normally obtained during a corresponding portion of the fermentation process, under the same conditions and for the same input values, except for the means causing the elevated ethanol concentration. The elevated ethanol concentration may be reached by achieving a surplus ethanol concentration obtained by e.g. direct addition to an ongoing fermentation process, or to a feed to be subsequently used in a fermentation process, of ethanol, or of an ethanol containing fluid having a higher ethanol content than the broth in the fermentation system without such additions, causing the broth being processed to have a higher ethanol concentration during the remainder of the fermentation process than without this addition. The elevated ethanol concentration may alternatively be reached by direct addition to an ongoing fermentation process, or to a feed subsequently to be used in a fermentation process, of a compound that is converted to ethanol "in situ" causing the broth being processed in the fermentation system to for at least some of the remainder part of the fermentation process to contain a higher ethanol content, than the broth otherwise would have at the corresponding part of the fermentation without this addition.

According to at least one example embodiment, the step of co-fermenting the fermentation media by means of fermentation microorganisms of yeast in the presence of an increased or elevated ethanol concentration is carried out for a sub-part of the fermentation process.

Hereby, the fermentation process can be divided into sub-parts, in which at least one sub-part comprises co-fermentation of the fermentation media in the presence of an increased or elevated ethanol concentration. Each sub-part of the fermentation process may be performed in a separate fermentation unit, e.g. a fermentation vessel. According to at least one example embodiment, the method further comprises the step of co-fermenting the fermentation media by means of fermentation microorganisms of yeast in the presence of a normal ethanol concentration for another sub-part of the fermentation process. Thus, the various sub-parts of the fermentation process can be combined in such a way that the overall fermentation rate is optimized. For example, during a first sub-part being e.g. an initial sub-part of the fermentation process, the fermentation media is co-fermented in the presence of a normal ethanol concentration, wherein subsequently, after a certain amount of the fermentation media has been converted, during a second sub-part, the fermentation media is co-fermented in the presence of a an increased or elevated ethanol concentration. The normal ethanol concentration is thus referring to a case in which the ethanol concentration has not been intentionally increased. The increased or elevated ethanol concentration is thus referring to a case in which the ethanol concentration is higher compared to what would normally be produced by the fermentable sugars in the fermentation media.

According to at least one example embodiment, the elevated ethanol concentration is 25 % higher compared to what would normally be produced by the fermentable sugars in the fermentation media. For example, the elevated ethanol concentration is an ethanol concentration totaling above that typically achieved by fermenting the initially available fermentable sugars in the fermentation media at 25 wt% or higher sugar to ethanol conversion/yield. According to another example, the increased or elevated ethanol concentration is an ethanol concentration totaling between 6 and 9 wt%.

According to at least one example embodiment, the elevated ethanol concentration is at least 2 wt%, or at least 50 % of the theoretical yield of ethanol obtainable by the used fermentation media.

For example, the elevated ethanol concentration is at least 2.5 wt%, or 3 wt%, or 4 wt%, or 5 wt% or 6 wt% or 7 wt% or 8 wt%, or at least 10 wt %, with an upper limit of e.g. 20 wt%, such that the elevated ethanol concentration is between 6 wt% (or 8 wt % or 10 wt%) and 20 wt %. Alternatively, the elevated ethanol concentration is at least 50 % - 80 % of the theoretical yield of ethanol obtainable by the used fermentation media.

According to at least one example embodiment, the ethanol concentration is denoted X g/L, and the amount of yeast is denoted Y g/L, wherein the yeast stress ratio is defined as X/Y, wherein X/Y is at least 10.

According to at least one example embodiment, the elevated ethanol concentration is reached by the external addition of ethanol, or ethanol producing substance corresponding to the same amount of ethanol, of between 10 g/l to 50 g/l, such as e.g. between 10 g/l to 20g/l or 30 g/l, or 40 g/l, or between 20 g/l to 30 g/l or 40 g/l or 50 g/l.

According to at least one example embodiment, the method further comprises the steps of:
- determining the ethanol concentration in the medium during the step of co-fermenting,
- in response of determining that the concentration of ethanol is below a threshold limit, adjusting the ethanol concentration to be above the threshold limit.

Hereby, an efficient means for adjusting the concentration of ethanol is provided, so that the desired ethanol concentration is maintained during fermenting. For example, the ethanol concentration is measured directly during the step of co-fermenting, e.g. based on an online measurement of the fermentation slurry comprising the fermentation media, the yeast, and any produced fermented product. Hereby, the delay between measuring the ethanol concentration and adjusting the ethanol concentration in response to the measurement, is minimized. Alternatively, the ethanol concentration is measured in an off-gas from the fermenting slurry.

According to at least one example embodiment, the step of co-fermenting is carried out in at least two separate fermentation units. Hereby, the co-fermentation can be better controlled, and the previously discussed sub-steps implemented by the separate fermentation vessels.

Thus, the method may be referred to increasing the fermentation rate of at least part of a microbial fermentation process, or a series of fermentation sub-processes carried out in separate fermentation units, so that the total fermentation processing time of one or the series of fermentation sub-processes is reduced.

According to at least one example embodiment, the yeast is a xylose-engineered yeast.

Thus, the yeast is configured to ferment xylose, and possibly other pentoses in addition to hexoses such as mannose and glucose. Hereby, the overall yield of the fermentation product may be increased. According to at least one example embodiment, the yeast is not Saccharomyces cerevisiae (S. cerevisiae), e.g. baker's yeast. However, according to at least one example embodiment, the yeast is a S. cerevisiae species that have been genetically engineered to be able to ferment one or more pentose sugars, such as xylose. According to at least one examples, the yeast is as any yeast strain belonging to the Saccharomyces genus (e.g. Saccharomyces cerevisiae, Saccharomyces bayanus, Saccharomyces uvarum). Yeast may refer to any organism belonging to the family Saccharomycetaceae, which include all the budding yeasts, e.g. also the Pichia genus.

According to at least one example embodiment, one of the first and second lignocellulosic biomass-based streams comprises at least glucose and xylose and are derived from e.g. hardwood, and the other one of the first and second lignocellulosic biomass-based streams comprises at least mannose and are derived from e.g. softwood, such as spruce or pine.

Hereby, a mixture of different lignocellulosic biomass materials can be used to reach the target range of the xylose to mannose ratio in the fermentation media.

According to a second aspect of the present invention, use of mannose to increase the fermentation rate of a microbial fermentation process involving co-fermentation of a fermentation media comprising at least glucose and xylose, wherein the amount of mannose is adapted to reach a ratio of xylose to mannose of the fermentation media within a target range, is provided.

Effects and features of this second aspect of the invention are largely analogous to those described above in connection with the first aspect of the invention. Embodiments mentioned in relation to the first aspect of the invention are largely compatible with the second aspect of the invention, of which some are exemplified below (typically without repeating the advantageous effects again).

According to at least one example embodiment, the target range is based on an increased fermentation rate during at least a part of the co-fermentation of the fermentation media.

Thus, as described in relation to the first aspect of the invention, the specific relative amount of mannose to xylose in the fermentation media, resulting in that the fermentation, or fermentation rate, of xylose is increased during at least a part of the co-fermentation, may be predefined, or determined in advance. That is, by knowing the preferred relative amounts of xylose and mannose in relation to the fermentation rate, and defining such preferred relative amounts as a predefined target range of the ratio of xylose to mannose, the desired improvement in the fermentation may be achieved. According to at least one example embodiment, the part of the co-fermentation of the fermentation media for which the predefined target range causes an increased fermentation rate comprises the change in fermentation tendency from glucose to xylose.

According to a third aspect of the invention, a system for increasing the fermentability of a microbial fermentation process involving co-fermentation of sugars from lignocellulosic biomass to fermentation products is provided. The system comprises:
- a fermentation unit configured to ferment a fermentation media comprising at least glucose, xylose and mannose;
- means of controlling that a ratio of xylose to mannose of the fermentation media is within a target range.

Effects and features of this third aspect of the invention are largely analogous to those described above in connection with the first aspect of the invention. Embodiments mentioned in relation to the first aspect of the invention are largely compatible with the third aspect of the invention, of which some are exemplified below (typically without repeating the advantageous effects again).

According to at least one example embodiment, the fermentation media is achieved as described in relation to the first aspect of the invention. Thus, the fermentation media is produced by:
- providing a first lignocellulosic biomass-based stream and a second lignocellulosic biomass-based stream different to the first lignocellulosic biomass-based stream, the first and second lignocellulosic biomass-based streams together comprising xylose, glucose and mannose;
- mixing the first and second lignocellulosic biomass-based streams;
- treating the mixed first and second lignocellulosic biomass-based streams, or treating the first and second lignocellulosic biomass-based streams separately prior to the step of mixing, to provide a fermentation media comprising xylose, glucose and mannose, wherein the mixing of the first and second lignocellulosic biomass-based streams results in a ratio of xylose to mannose in the fermentation media within a target range;

The means of controlling that the ratio of xylose to mannose of the fermentation media is within a target range depends on where in the the process of from providing lignocellulosic biomass raw material, to prior to fermentation, the mixing of the first and second lignocellulosic biomass-based streams is carried out. The means may e.g. comprise a mixing device for mixing the first and second lignocellulosic biomass-based streams, e.g. configured to mix of the first and second lignocellulosic biomass-based streams prior to pretreating the lignocellulosic biomass, i.e. subjecting the lignocellulosic biomass to a pretreatment process, or configured to mix the first and second lignocellulosic biomass-based streams subsequent to the pretreatment process, and e.g. prior a hydrolysis step of the pretreated lignocellulosic biomass, or configured to mix the first and second lignocellulosic biomass-based streams subsequent to the hydrolysis step, but prior to any fermentation step.

For a continuous, or semi-continuous, fermentation process, the means may further comprise a measuring device configured to measure a residual sugar indicator parameter RSI, which parameter directly or indirectly indicates the concentration of residual sugars, during fermentation in the fermentation vessel, and means for determining an RSI setpoint based on a rate of change of the measured residual sugar indicator parameter, such that the RSI setpoint corresponds to a maximum rate of change; and means to automatically adjust the amount of fermentable sugars of glucose, mannose and xylose in the fermentation media in response to the measured residual sugar indicator parameter RSI and the RSI setpoint, so as to achieve and maintain the RSI setpoint, whereby efficient co-fermentation of sugars to fermentation products is obtained. For example, the adaptation of the amount of fermentable sugars of glucose, mannose and xylose in the fermentation media may be achieved by adapting the first and/or the second lignocellulosic biomass-based streams. The RSI setpoint is preferably within the changeover phase as previously described, or is adapted to be within the fermentation phase in which xylose is being co-fermented (simultaneously) with the mannose (or any hexose).

According to at least one example embodiment, the system comprises control means of controlling the concentration of ethanol present in the fermentation unit during the fermentation to an intentionally increased or elevated ethanol concentration, wherein the means is configured to reach the ethanol concentration by the addition of ethanol or an ethanol producing substance which is converted into ethanol in situ. The control means may e.g. be a control arrangement. The control arrangement may comprise adequate equipment, such as e.g. a controllable valve connected to a supply unit comprising ethanol or an ethanol-producing substance, which controllable valve is arranged on a supply line to, or upstream of, the fermentation unit, and is configured to vary the supply of ethanol or ethanol-producing substance in response of determining that the concentration of ethanol is below a threshold limit by means of the ethanol measurement arrangement.

According to at least one example embodiment, the system and the fermentation unit is configured to carry out the fermentation as a continuous fermentation process.

According to at least one example embodiment, the fermentation unit is a first fermentation unit and the system further comprises a second fermentation unit, the first and second fermentation units being configured to subsequently ferment the fermentation media. The fermentation unit(s) may be fermentation vessel(s).

The system may comprise additional units and components known to those skilled in the art. For example, a pretreatment arranged for pretreating the lignocellulosic biomass, a hydrolysis unit for hydrolyzing the pretreated lignocellulosic biomass, and/or a separation unit arranged between the pretreatment arrangement and the hydrolysis unit, and/or between the hydrolysis unit and the fermentation unit.

### BRIEF DESCRIPTION OF THE DRAWINGS

The various aspects of the present invention, including its particular features and advantages, will be readily understood from the following detailed description and the accompanying drawings, in which:
Fig. 1 schematically illustrates an arrangement for treatment of lignocellulosic biomass including control of a microbial fermentation process in accordance with example embodiments of the invention, and
Fig. 2 schematically illustrates a system for increasing the fermentability of a microbial fermentation process in accordance with example embodiments of the invention, and
Fig. 3 schematically illustrates the steps of a method of increasing the fermentability of a microbial fermentation process in accordance with one example embodiment of the invention.
Fig. 4 is a graph showing the results of a first series with four measurement trials in accordance with one example embodiment of the invention;
Fig. 5a is a graph showing the results of a second series with four measurement trials in accordance with example embodiments of the invention;
Figs. 5b and 5c are enlarged views of different parts of the graph in Fig. 5a, in accordance with one example embodiment of the invention;
Fig. 6a is a graph showing the results of a third series with three measurement trials in accordance with one example embodiment of the invention;
Figs. 6b, 6c and 6d are enlarged views of different parts of the graph in Fig. 6a, in accordance with example embodiments of the invention; and
Figs. 7a and 7b are graphs showing the results of a fourth and fifth series with a respective four measurement trials in accordance with example embodiments of the invention; and

### DETAILED DESCRIPTION

The present invention will now be described more fully hereinafter with reference to the accompanying drawings. The present invention may, however, be embodied in many different forms and should not be construed as limited to the embodiments set forth herein; rather, these embodiments are provided for thoroughness and completeness, and fully convey the scope of the present invention to the skilled person.

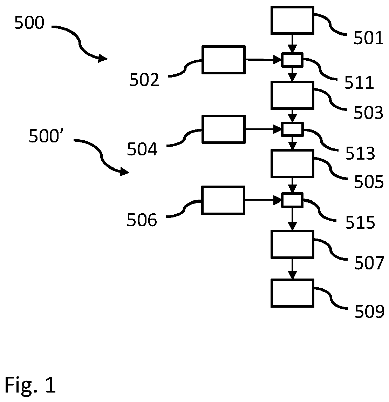

With reference to Fig. 1, the present disclosure provides an arrangement 500 for treatment of lignocellulosic biomass comprising a supply unit 501 for supplying the lignocellulosic biomass as a raw material, a pretreatment arrangement 503 for pre-treating the lignocellulosic biomass, a hydrolysis unit 505 in which the pretreated biomass is subject to enzymatic hydrolysis by means of saccharification enzymes, the hydrolysis unit 505 being arranged downstream of and in fluid communication with the pretreatment arrangement 503, and comprises a fermentation unit 507, such as a fermentation vessel 507, arranged downstream of and in fluid communication with the hydrolysis unit 505. In the fermentation vessel 507, the hydrolysate, or fermentation media, comprising fermentable sugars is fermented into a target chemical, e.g. ethanol, by means of a yeast. The arrangement 500 may also comprise a product recovery unit 509, such as distillation or ion exchange chromatography, arranged downstream of and in fluid communication with the fermentation vessel 507.

In Fig. 1, the fermentation unit 507 is comprised in a system 500' for increasing the fermentability of the microbial fermentation process by controlling a ratio of specific sugars in the fermentation media. In more detail, the fermentation media in the fermentation unit 507 is produced by providing a first lignocellulosic biomass-based stream and a second lignocellulosic biomass-based stream, and mixing the first and second lignocellulosic biomass-based streams. The second lignocellulosic biomass-based stream is different to the first lignocellulosic biomass-based stream, and the first and second lignocellulosic biomass-based streams together comprises at least the three fermentable sugars xylose, glucose and mannose. The mixing of the first and second lignocellulosic biomass-based streams may be performed in various ways depending on where in the the process of from providing lignocellulosic biomass raw material, e.g. by supply unit 501, to prior the fermentation process in the fermentation unit 507, the mixing of the first and second lignocellulosic biomass-based streams is carried out.

In Fig. 1, three potentials process positions of the mixing are shown, of which only one is needed. A first mixing position is arranged downstream of the supply unit 501 and upstream of the pretreatment arrangement 503, in which the first lignocellulosic biomass-based stream is provided by the supply unit 501 as a first raw material, and the second lignocellulosic biomass-based stream is provided by a secondary supply unit 502 as a second raw material. For example, the first raw material may be hardwood comprising at least glucose and xylose, and the second raw material may be softwood comprising at least mannose. The first and second raw materials may be mixed in a first mixing device 511, the first mixing device 511 being e.g. a container or screw feeder. A second mixing position is arranged downstream of the pretreatment arrangement 503 and upstream of the hydrolysis unit 505, in which the first lignocellulosic biomass-based stream is provided by the pretreatment arrangement 503 as a first pretreated lignocellulosic biomass slurry, and the second lignocellulosic biomass-based stream is provided by a secondary pretreatment arrangement 504 as a second pretreated lignocellulosic biomass slurry. For example, the first pretreated lignocellulosic biomass slurry may be derived from hardwood and comprising at least glucose and xylose, and the second pretreated lignocellulosic biomass slurry may be derived from softwood and comprising at least mannose. The first and second lignocellulosic biomass slurries may be mixed in a second mixing device 513, the second mixing device 513 being e.g. a container or screw feeder. A third mixing position is arranged downstream of the hydrolysis unit 505 and upstream of the fermentation unit 507, in which the first lignocellulosic biomass-based stream is provided by the hydrolysis unit 505 as a first hydrolysate, and the second lignocellulosic biomass-based stream is provided by a secondary hydrolysis unit 506 as a second hydrolysate. For example, the first hydrolysate may be derived from hardwood and comprising at least glucose and xylose, and the second hydrolysate may be derived from softwood and comprising at least mannose. The first and second hydrolysates may be mixed in a third mixing device 515, the third mixing device 515 being e.g. a container or screw feeder.

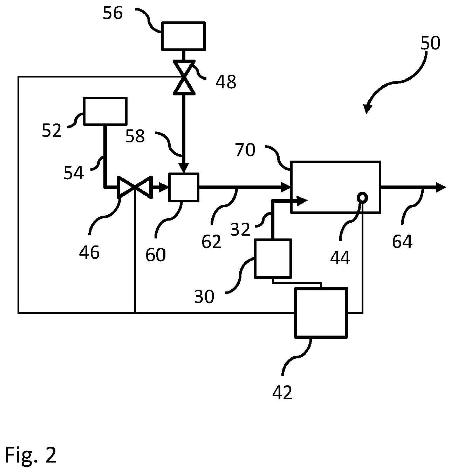

By providing a first and second lignocellulosic biomass-based streams with known characteristics and content of xylose, mannose and glucose, and mixing the first and second lignocellulosic biomass-based streams, the ratio of xylose to mannose may be controlled to be within a target range in the fermentation media. Thus, the first, second and/or third mixing devices 511, 513, 515 may be considered means of controlling that the ratio of xylose to mannose of the fermentation media is within the target range. Thus, the previously mentioned system 500' may comprise the fermentation unit 507 and any one of the first, second and third mixing device 511, 513, 515. An example of such system 500' will now be described with reference to Fig. 2.

Fig. 2 schematically a system 50 for increasing the fermentability of a microbial fermentation process involving co-fermentation of sugars from lignocellulosic biomass to fermentation products. The system 50 comprises means 52 for providing a first lignocellulosic biomass-based stream 54, the means 50 being e.g. a supply line from an upstream hydrolysis unit (as hydrolysis unit 505 of Fig. 1) or a moisture adjusting unit configured to intentionally increase or decrease the concentration of sugars in the first lignocellulosic biomass-based stream 54. The system further comprises means 56 for providing a second lignocellulosic biomass-based stream 58, the means 56 being e.g. a supply line from an upstream hydrolysis unit (as secondary hydrolysis unit 506 of Fig. 1) or a moisture adjusting unit configured to intentionally increase or decrease the concentration of sugars in the second lignocellulosic biomass-based stream 58. The first and the second lignocellulosic biomass-based streams 54, 58 together comprises at least three fermentable sugars being xylose, mannose and glucose. Moreover, the system 50 comprises a mixing device 60 configured to mix the first and the second lignocellulosic biomass-based streams 54, 58 to provide a fermentation media 62, and a fermentation unit 70 configured to ferment the fermentation media 62.

The system 50, and the fermentation unit 70, in Fig. 2 are configured to continuously ferment the fermentation media 60 by co-fermenting the fermentable sugars, at least xylose, mannose and glucose, by means of fermentation microorganisms of yeast, to a fermentation product 64. Moreover, the system 50 comprises a measuring and control device 42 configured to measure a residual sugar indicator parameter RSI of the fermentation media (or fermentation slurry) of the fermentation unit 70. In Fig 2, the measuring and control device 42 comprises a measuring probe 44 being in direct contact with the fermentation media (or fermentation slurry) in the fermentation unit 70 for an online measurement of the RSI parameter, to directly indicate the concentration of residual sugars during fermentation in the fermentation unit 70. The measuring and control unit 42 may be configured to determine an RSI setpoint based on a rate of change of the measured RSI parameter, such that the RSI setpoint corresponds to a maximum rate of change, and may automatically adapt the amount of fermentable sugars of glucose, mannose and xylose in the fermentation media 62 in response to the measured RSI parameter and the RSI setpoint. For example, the adaptation of the amount of fermentable sugars of glucose, mannose and xylose in the fermentation media 62 may be achieved by adapting the first and/or the second lignocellulosic biomass-based streams 54, 58. This may e.g. be achieved by that the measuring and control device 42 controls a first valve 46 varying the amount of the first lignocellulosic biomass-based stream 54 to the mixing device 60 and/or a second valve 48 varying the amount of the first lignocellulosic biomass-based stream 54 to the mixing device 60. Alternatively, the measuring and control device 42 may control the actual mixing in the mixing device 60. It should be understood that when adapting the amount of fermentable sugars of glucose, mannose and xylose in the fermentation media 62 by adapting the first and/or the second lignocellulosic biomass-based streams 54, 58, the ratio of xylose to mannose is known in a predetermined manner, and is kept within the target range. Thus, a means of controlling the ratio of xylose to mannose of the fermentation media 46 is within a target range is provided by the first and second valves 46, 48 and the provision of mixing a first and a second lignocellulosic biomass-based streams comprising xylose, mannose and glucose in the mixing device 60.

According to at least one example embodiment, the measuring and control unit 42 may be configured to automatically adapt the ratio of xylose to mannose such that the target range of the xylose to mannose ratio is reached in the fermentation media as previously described, in response to the measured residual sugar indicator parameter RSI and the RSI setpoint. Thus, the first and/or the second lignocellulosic biomass-based streams 54, 58, may be directly controlled and adapted via the first and second valves 46, 48 to adapt the ratio of xylose to mannose. For example, an HPLC (high-performance liquid chromatography) may be used to determine the concentration of various sugars in the fermentation unit, and/or the first and/or the second lignocellulosic biomass-based streams 54, 58. Moreover, for such embodiments, the mixing device 60 may be omitted, and the first and second lignocellulosic biomass-based streams 54, 58 directly fed to the fermentation unit 70.

Moreover, the system 50 may comprises a supply unit 30 configured to supply external ethanol and/or an external ethanol-producing substance, such as external sugars, e.g. glucose, and a supply line 32 connecting the supply unit 30 with the fermentation unit 70. Alternatively, any one of the means for providing the first and second lignocellulosic biomass-based streams 52, 56 may be a dewatering unit for intentionally varying the amount of fermentable sugars to intentionally increase the ethanol concentration in the fermentation unit 70. Thus, the supply unit 30 and supply line 32, as well as any dewatering unit as the means 52, 56, constitute different means of intentionally increasing the concentration of ethanol present in the fermentation unit 70 during operation (i.e. during the fermentation) to an elevated ethanol concentration. For example, by increasing the concentration of sugars by means of a dewatering unit, an increased amount of ethanol will be produced in the fermentation unit 70. However, it should be noted that the system 50 needs not to be provided with both a dewatering unit and the supply unit 30 with the supply line 32, but any means for intentionally increasing the concentration of ethanol to an elevated ethanol concentration is sufficient. Thus, by a dewatering unit, and/or the supply unit 30 with the supple line 32, it is possible to reach the elevated ethanol concentration by the addition of ethanol or an ethanol-producing substance in the fermentation unit 70. Other means for intentionally increasing the ethanol concentration to the elevated ethanol concentration is by the addition of external ethanol or external sugars upstream of the fermentation unit 70 (not shown). By adding an ethanol producing substance, such as sugars or glucose, and by operating the fermentation unit 70 in a continuous manner to in situ produce ethanol, the elevated ethanol concentration may be maintained in the fermentation unit 70 during the fermentation. The elevated ethanol concentration is believed to result in that the yeast ferment the xylose and glucose more equally compared to normal conditions, i.e. conditions in which the ethanol concentration is not intentionally increased to the elevated ethanol concentration. Thus, by the elevated ethanol concentration, the fermentation of xylose relative the fermentation of glucose is increased. In other words, the relative fermentation rate of xylose to the fermentation rate of glucose is increased.

The elevated ethanol concentration in the fermentation unit 70 may be controlled by the measuring and control device 42. Alternatively, a separate control unit (not shown) may be used to control the ethanol concentration of the fermentation unit 70. The measuring probe 44 may be used to measure the ethanol concentration in the fermentation unit 70 alternatively, or in addition to, the RSI parameter. Thus, the measuring probe 44, the measuring and control device 42, and the supply unit 30 with the supply line 32 may thus form an automatically ethanol adjusting arrangement configured to measure the ethanol concentration in the fermentation unit 70 and automatically adjust the ethanol concentration in the fermentation unit 70 by means of supply of external ethanol and/or external sugars. It should be noted that the system 50 may comprise a secondary fermentation unit (not shown) arranged downstream of the fermentation unit 70.

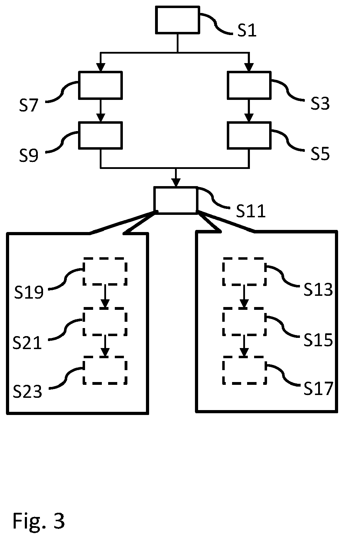

The invention will now be described with reference to the flow chart of Fig. 3 which e.g. includes the operation of the system 50 of Fig. 2. The flow chart of Fig. 3 discloses the steps of a method of increasing the fermentability of a microbial fermentation process involving co-fermentation of sugars from lignocellulosic biomass to fermentation products.

In a step S1, being e.g. a first step S1, a first lignocellulosic biomass-based stream and a second lignocellulosic biomass-based stream different to the first lignocellulosic biomass-based stream are provided. The first and second lignocellulosic biomass-based streams together comprises xylose, glucose and mannose. The first and second lignocellulosic biomass-based streams may be derived from hardwood, softwood, agricultural waste or any mixture thereof. According to one example embodiment, one of the first and second lignocellulosic biomass-based streams comprises at least glucose and xylose (e.g. derived from hardwood), and the other one of the first and second lignocellulosic biomass-based streams comprises at least mannose (e.g. derived from softwood, such as spruce or pine).

In a step S3, being e.g. a second step S3, the first and second lignocellulosic biomass-based streams are mixed. For example, the first and second lignocellulosic biomass-based streams are mixed to reach a ratio of xylose to mannose within a target range.

In a step S5, being e.g. a third step S5, the mixed first and second lignocellulosic biomass-based streams are treated to provide a fermentation media comprising xylose, glucose and mannose.

In an alternative embodiment, the step S3 and S5 are swopped so that each one of the separate first and second lignocellulosic biomass-based streams are treated, shown as an alternative step S7, prior to a step of mixing the first and second lignocellulosic biomass-based streams, shown as an alternative step S9.

Treating the mixed first and second lignocellulosic biomass-based streams, or the separate first and second lignocellulosic biomass-based streams may include at least one of the following actions: pretreating (e.g. in a pretreatment arrangement 503 described with reference to Fig. 1), hydrolyzing (e.g. in a hydrolyzing unit 505 described with reference to Fig. 1), addition of nutrients, adjustment of pH. For the separate pre-treatment and hydrolysis of the first and second lignocellulosic biomass-based streams, treating may simply be referred to as mixing and/or transporting and/or temperature controlling the first and second lignocellulosic biomass-based streams.

In either way, the result of the steps of treating and mixing, S3, S5, S7, S9, is to provide a fermentation media comprising xylose, glucose and mannose, with a ratio of xylose to mannose in the fermentation media within a target range. The target range is different to a ratio of xylose to mannose in the separate first and/or second lignocellulosic biomass-based streams. The target range may e.g. extend from 0.08 to 13. Such target range may be based on the respective concentrations of xylose and mannose, or the respective amount of xylose and mannose to the total amount of fermentable sugars in the fermentation media, or to the total amount of xylose, mannose and glucose in the fermentation media. Stated differently, the amount of xylose in the fermentation media may be between 5 and 65 wt%, and the amount of mannose in the fermentation media may be between 5 and 65 wt%, as compared to the total amount of glucose, mannose and xylose.

In a step S11, being e.g. fourth step, the fermentation media is co-fermented by means of fermentation microorganisms of yeast to a fermentation product. This may e.g. be carried out by a fermentation unit (as fermentation unit 507 of Fig. 1). The yeast is preferably a xylose -engineered yeast, i.e. a yeast with the capability of fermenting xylose (which typically is carried out when any glucose in the fermentation media is exhausted, or is close to be exhausted).

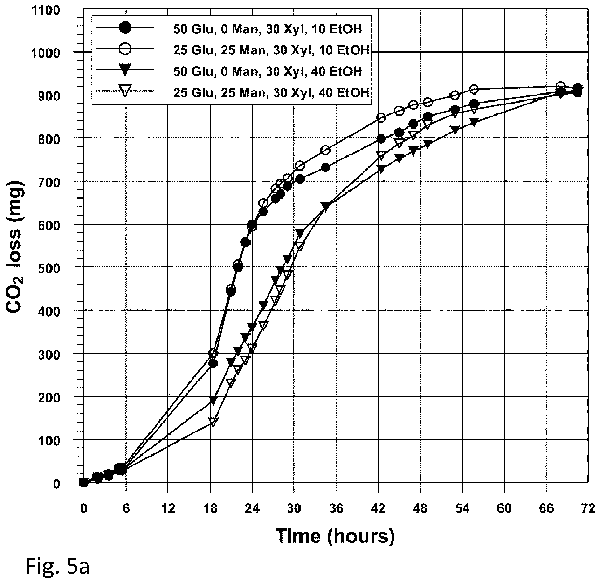

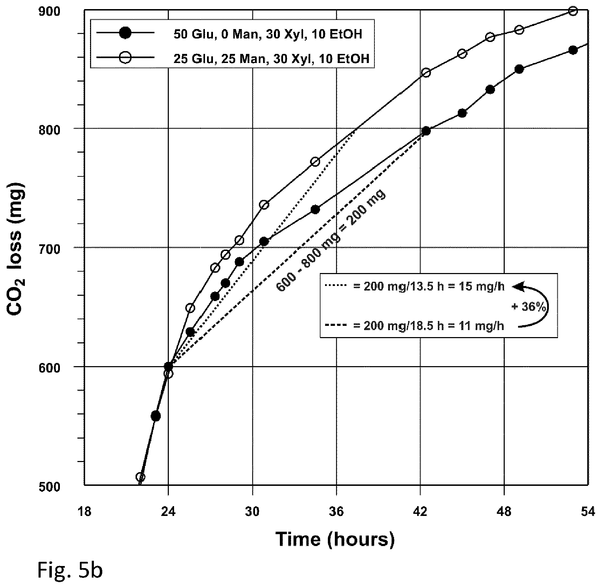

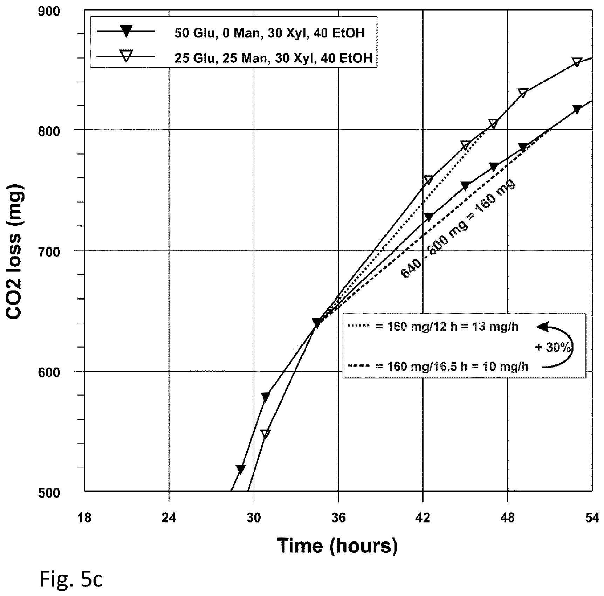

The target range is a preferably a predefined target range, the predefined target range being based on an increased fermentation rate during at least a part of the co-fermentation S11 of the fermentation media. Thus, by providing a fermentation media with a ratio of xylose to mannose within a target range, the fermentation may be improved, and the fermentation rate increased for at least a part of the co-fermentation S11. Such part of the co-fermentation S11 of the fermentation media preferably comprises the change in fermentation substrate or fermentation tendency from glucose to xylose, as will be further described with reference to e.g. Fig. 5.

Preferably, the step of co-fermenting S11 is performed in a continuous, or semi-continuous, manner in a fermentation vessel. For such embodiments, the step of co-fermenting S11 may be performed while the following steps are carried out.

In a sub-step S13, being e.g. a first sub-step S13, a residual sugar indicator parameter RSI, which parameter directly or indirectly indicates the concentration of residual sugars, is measured during the fermentation process in the fermentation vessel.

In a sub-step S15, being e.g. a second sub-step S15, an RSI setpoint based on a rate of change of the measured residual sugar indicator parameter, is determined such that the RSI setpoint corresponds to a maximum rate of change.

In a sub-step S17, being e.g. a third sub-step S17, the amount of fermentable sugars of glucose, mannose and xylose in the fermentation media is adapted in response to the measured residual sugar indicator parameter RSI and the RSI setpoint, so as to achieve and maintain the RSI setpoint, whereby efficient co-fermentation of sugars to fermentation products is obtained. During such adaption of the amount of fermentable sugars of glucose, mannose and xylose, the ratio of xylose to mannose is adapted to be kept within the target range (by knowing the content of the mannose, xylose and glucose in the combined or mixed first and second lignocellulosic biomass-based streams).

According to at least one example embodiment, the method may comprise the step S19 of intentionally increasing the ethanol concentration of the fermentation media, and performing the step of co-fermenting S11 of the fermentation media in the presence of an intentionally increased ethanol concentration of at least 2 wt%. For such embodiments, the method may further comprise the step S21 of determining the ethanol concentration in the medium during the step of co-fermenting, and in response of determining that the concentration of ethanol is below a threshold limit, the step S23 of adjusting the ethanol concentration to be above the threshold limit. In Fig. 3, optional steps are indicated by dashed line boxes.

The system 50 in Fig. 2 is configured to carry out the fermentation as a continuous fermentation process in the fermentation unit 70, but it may as well be configured as a batch fermentation process in which the fermentation media is fermented batch-wise. For embodiments in which the method is automated and adapted for continuous operation, the method may, alternatively or in addition to the target range of the ratio of xylose to mannose, and the ethanol concertation, comprise a step of collecting and monitoring various process parameters in a continuous or semi-continuous manner and a step of automatically adjusting the fermenting process in response to the monitored process parameters.

### EXAMPLES

Co-fermentation of different fermentation media including various amounts of the three sugars xylose, mannose and glucose, were performed batch-wise in closed vessels equipped with a canula for venting the generated CO2 and containing 25 ml fermentation media of the various sugar amounts, water and, using a standard medium comprising nutrients such as nitrogen, minerals and vitamins for yeast growth and fermentation. The pH was adjusted to 5.5 with NaOH .

Inoculation was performed with freshly hydrated active dry yeast at 1 or 2 g/L DW amount. Where ethanol was added, it was added after 4-6 hours of inoculation to let the cells initiate the metabolism after the lag phase. This has proved to be necessary when adding the indicated amounts of ethanol to the cultures. The temperature was kept at 30 °C, and the vessels were shaken on a rotary shaker with a speed just sufficiently to keep the yeast in suspension. This type of fermentation media preparation was performed for all measurement trials below, and the results are presented as fermentation process and fermentation rates as weight loss values, which indicates total amount of generated CO₂ vented through the canula, equivalent of ethanol generated.

Five different series including various measurement trials or samples, were carried out for sugar mixtures with various amounts of xylose, mannose and glucose. For some samples, ethanol was added as described above.

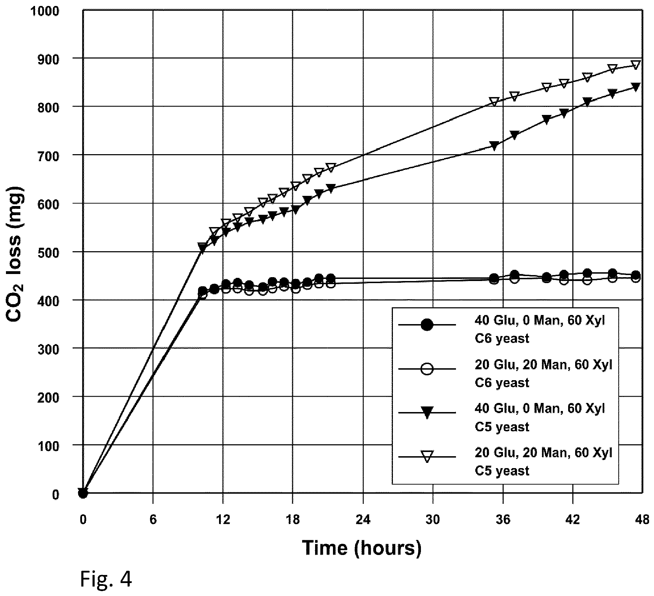

In the first series, four samples were carried out as shown in Fig. 4. Two pairwise samples, with either 40 g/L glucose and 60 g/L xylose, or 20 g/L glucose, 20 g/L mannose and 60 g/L xylose, each pair inoculated with either a C5 yeast or a C6 yeast, were conducted. The C5 yeast refers to a yeast configured to ferment pentoses (and hexoses), being any microorganism that is a member of the saccharomyces genus, and that has been engineered to be able to ferment one or more pentose sugars in addition to hexose sugars, and the C6 yeast refers to a yeast configured to ferment hexoses (but not pentoses), thus being any microorganism that is a member of the saccharomyces genus, and that is naturally limited in fermentation ability to ferment only hexose sugars. For example, the C5 yeast is a yeast of the S. cerevisiae species that have been genetically engineered to be able to ferment one or more pentose sugars, such as xylose, and the C6 yeast is a wild type yeast of the S. cerevisiae species that is able to ferment hexose sugars only. As seen in Fig. 4, when using the C6 yeast, there is no discernible difference between the sample with only glucose and xylose, and the sample with half the glucose exchanged with mannose. Thus, the C6 yeast seems to ferment glucose and mannose at approximately the same rate. On the other hand, when using the C5 yeast, the fermentation proceeds after the theoretical hexose limit, evident when being compared with the C6 yeast fermentation samples. It is to be expected that the fermentation proceeds with xylose in a xylose fermenting phase after the hexoses have been exhausted. Surprisingly, the fermentation rate is higher in the xylose fermenting phase when part of the glucose has been exchanged with mannose compared to the sample comprising only glucose and xylose. Thus, in Fig. 4, at least two distinct phases of the fermentation process can be elucidated, the hexose fermentation phase of between 0-10 hours, and the xylose fermentation phase between 10 hours to the end points at 48 hours. During the hexose fermentation phase, mainly hexoses as glucose and mannose are fermented as the yeast typically favours hexoses over xylose (or pentoses). As less glucose and potentially mannose is present in the fermentation media, towards the end of the hexose fermentation phase, the tendency of fermentation is changed from hexose to pentose, as the yeast changes from fermenting glucose to fermenting xylose. This change, or changeover, from the hexose fermentation phase to the xylose fermentation phase may be referred to as a changeover phase, occurring 10-12 hours in Fig. 4.

Fig. 5a shows a second series with four curves based on data from two sample pairs with xylose concentration reduced to 30 g/L, and two different levels of additionally added ethanol (10 g/L and 40 g/L). The glucose and mannose concentrations are varied between 50 g/L and 25 g/L, and 0 and 25 g/L, respectively. The fermentation processes were carried out using a C5 yeast, and kept running until the fermentable sugars had been exhausted, thus confirming that the same amount of fermentable sugars was present from the beginning of all four samples. It was expected that the addition of the higher amount of ethanol would be inhibitory to the fermentation, which is clearly seen during the time period from 6 hours to 30 hours. Moreover, the samples with the higher ethanol concentration also indicate that mannose fermentation is inhibited to a higher extend than glucose fermentation. But still, during the xylose fermentation phase from around 30-36 hours to 54-72 hours, the samples that had half the glucose exchanged for mannose (i.e. 25 g/L glucose and 25 g/L mannose) in the fermentation media fermented faster in the xylose fermentation phase than the samples without mannose. The curves indicate that the fermentation rate difference starts somewhere at a CO2 loss of about 600 mg.

Fig. 5b is an enlarged view of a section of the graph in Fig. 5a extending between 500 and 900 mg CO2 loss showing the curves from the samples with 10 g/L ethanol, where the fermentation rate impact of mannose appears to be prominent. Fig. 5c is an enlarged view of a section of the graph in Fig. 5a extending from 500 and 900 mg CO2 loss showing the curves from the samples with 40 g/L ethanol. By comparing the elapsed time needed from 600 mg CO2 loss to 800 mg loss for the samples with 10 g/L ethanol added (Fig. 5b), and the elapsed time from 640 mg CO2 loss to 800 mg loss for the samples with 40 g/L ethanol added (Fig. 5c), the magnitude of the advantage can be seen. For the samples with 10 g/L ethanol added, the elapsed time from 600 mg CO2 loss to 800 mg/CO2 loss is 13.5 hours for the mannose containing sample (25 g/L), and 18.5 hours for the sample without mannose, corresponding to a rate increase in this fermentation phase of 37 %. For the samples with 40 g/L ethanol added, the elapsed time from 640 mg CO2 loss to 800 mg/CO2 loss is 12.5 hours for the mannose containing sample (25 g/L), and 16.5 hours for the sample without mannose, corresponding to a rate increase in this fermentation phase of 32 %.

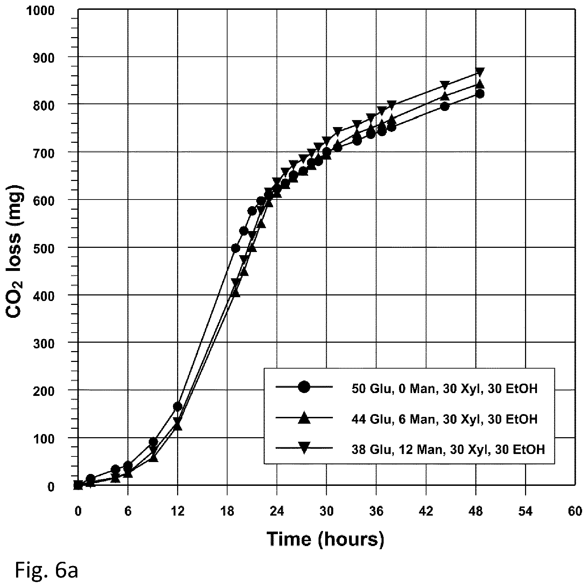

Fig. 6a shows a third series with three curves based on data from samples with a xylose concentration of 30 g/L and 30 g/L of added ethanol. The glucose and mannose concentrations were varied between 50 g/L, 44 g/L and 38 g/L, and 0, 6 g/L and 12 g/L, respectively. It is clear from Fig. 6a that even smaller additions of mannose have a fermentation rate enhancing effect in this particular fermentation phase. The effect is best seen in the fermentation phase where the CO2 loss goes from 500 mg to 630 mg when roughly 10 % and 20 % of the glucose has been substituted with mannose (i.e. the samples with 6 g/L mannose and 12 g/L mannose).

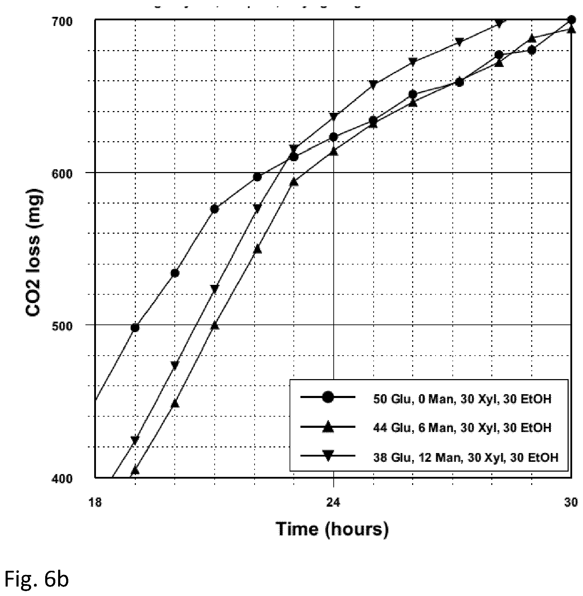

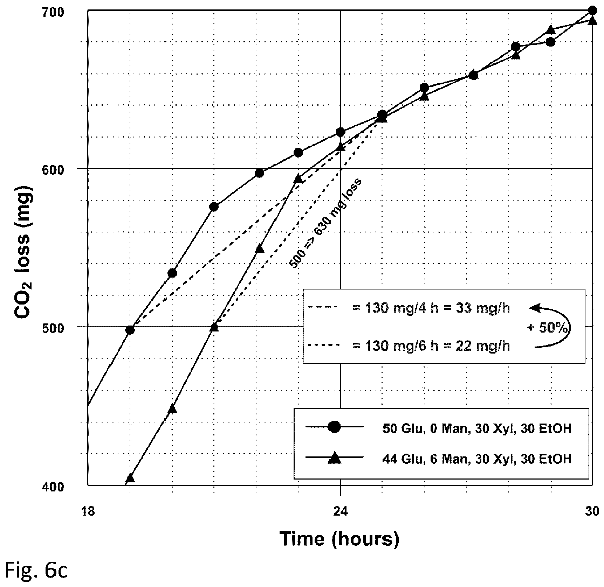

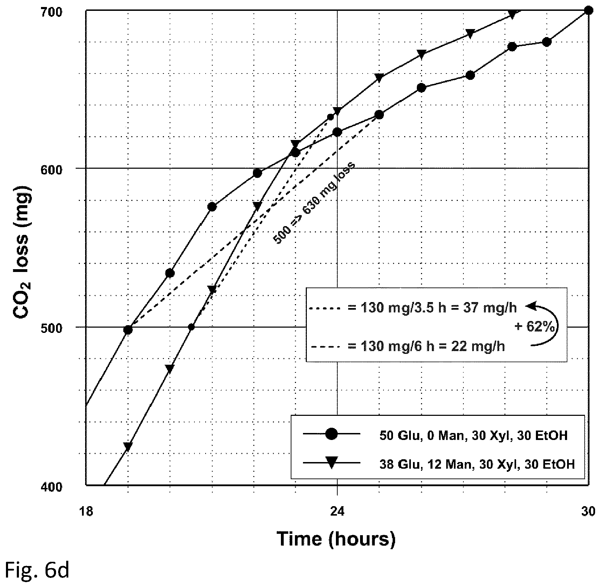

Fig. 6b is an enlarged view of a section of the graph in Fig. 6a extending between 400 and 700 mg CO2 loss. Fig. 6c is the same enlarged view showing only the curves from the samples with 0 mannose and 6 g/L mannose, and Fig. 6d is the same enlarged view showing only the curves from the samples with 0 mannose and 12 g/L mannose. By comparing the elapsed time needed from 500 mg CO2 loss to 630 mg loss for the sample with 0 mannose and the sample with 6 g/L mannose in Fig. 6c, a 50 % increase in the fermentation rate can be elucidated. For the corresponding samples of Fig. 6d, and comparing the elapsed time needed from 500 mg CO2 loss to 630 mg loss, for the sample with 0 mannose and the sample with 12 g/L mannose in Fig. 6d, a 62 % increase in the fermentation rate can be elucidated.

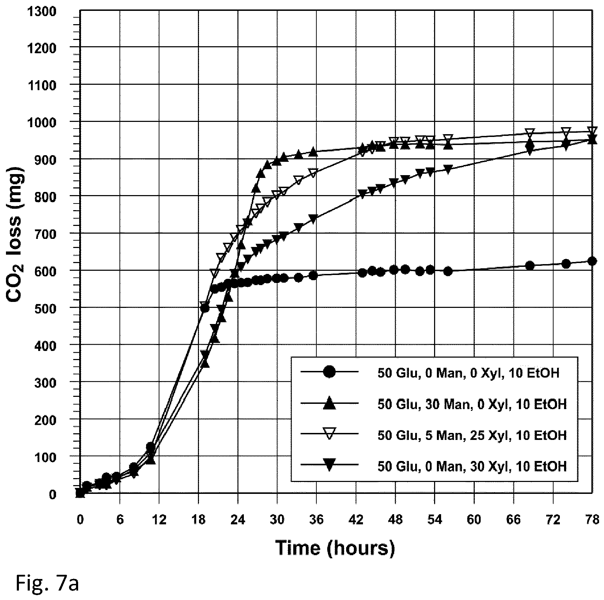

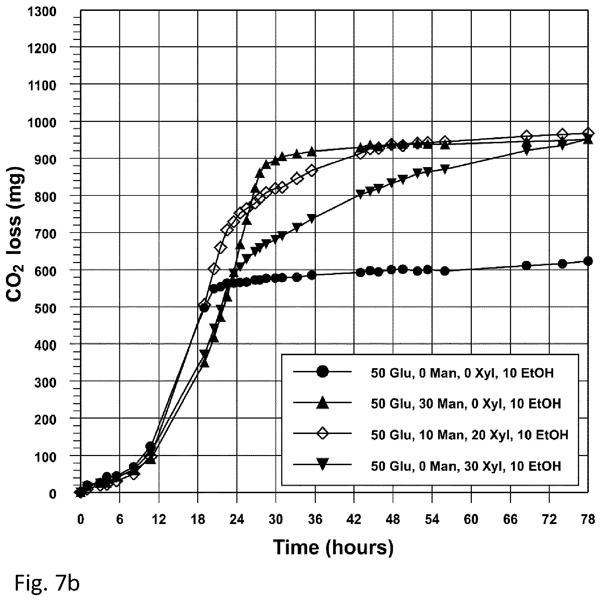

Figs. 7a and 7b show a fourth respective a fifth series, each with four curves based on data from samples with a glucose concentration of 50 g/L and 10 g/L of added ethanol. The xylose and mannose concentrations were varied between 0 g/L, 25 g/L and 30 g/L, and 0, 5 g/L and 30 g/L, respectively in Fig. 7a (fourth series), and between 0 g/L, 20 g/L and 30 g/L, and 0, 10 g/L and 30 g/L, respectively in Fig. 7b. It is clear from Figs. 7a and 7b that the advantage of mixing mannose and xylose containing feedstock can be realized as a saving in overall fermentation time. That is, the mannose effect can be seen by exchanging a minor part of the xylose with mannose, i.e. by comparing the sample with 5 g/L mannose and 25 g/L xylose in Fig. 7a with the sample with 10 g/L mannose and 20 g/L xylose in Fig. 7b. The mixing benefit can here be quantified as a reduced overall fermentation time for a total amount of 4 of different feeds.

In ethanol production, the batch fermentations may be terminated before the fermentable sugars have been completely exhausted, as the ethanol generation rate is falling below a chosen threshold when only a small fraction of the fermentable sugars remains. In the example of Figs. 7a and 7b, it appears reasonable to terminate the fermentation process when the CO2 loss (that corresponds to generated ethanol) reaches 940 mg. This threshold is reached at 48 hours for the sample without xylose, thus containing only glucose and mannose. Whereas the sample containing only glucose and xylose requires 78 hours fermentation time to reach that threshold. In Figs. 7a and 7b, we see that the exchange of 15 % or 30 % of the xylose with mannose in the glucose/xylose only media results in an improved fermentation, allowing these fermentation media to complete fermentation to the threshold also after 48 hours.

If assuming that the glucose-mannose and glucose-xylose samples in Fig. 7a are to be fermented in a fermentation unit subsequently to each other, by first fermenting two volumes of the samples with mannose and glucose (i.e. 50 g/L of glucose and 30 g/L of mannose with 10 g/L of added ethanol) for 2 ^{∗} 48 hours = 96 hours, and then fermenting two volumes of the sample with glucose and xylose (i.e. 50 g/L glucose, 30 g/L xylose with 10 g/L of added ethanol) for 2^{∗}78 hours, will result in a total fermentation time of 252 hours.

If instead using an advantageous mixing strategy with mannose and xylose, e.g. in which half of the glucose/mannose amount is mixed into the glucose/xylose part, providing three fermenter volumes of a 1:2 mixture, that corresponds to a 33 % substitution of xylose with mannose, the total fermentation time can be reduced for the same amount of sugars. By first fermenting the glucose/mannose media for 48 hours as previously described, and then ferment 3 ^{∗} the 1:2 mixtures as described above, each requiring 48 hours fermentation time, a total of 4 ^{∗} 48 hours which will result in a total fermentation time of 192 hours, saving a total of 60 hours, or about 25 % of the fermentation time compared to the previous example. The increased fermentation rate results in the benefit of achieving a higher fermentation capacity with fixed fermenter capacity if one goes from non-mixed/non-controlled fermentation to applying this invention.

According to at least one example embodiment, the fermentation process, or co-fermentation, may be divided into different fermentation units or fermentation vessels.

Even though the present disclosure has been described with reference to specific exemplifying embodiments thereof, many different alterations, modifications and the like will become apparent for those skilled in the art. For example, the batch-wise fermentation used in the examples above is for demonstrating the technical effect of the various sugar mixtures, and should not be regarded as limiting of the invention. Rather, the correlation of the results, and the use thereof, in a continuous fermentation process is of great advantage. A continuous fermentation process is of particular interest, as a desired setpoint (as the RSI setpoint described earlier) may be arranged to coincide with the fermentation phase where the rate advantage of mixing the various sugars is greatest. Returning to Fig. 4, were it is clear that the maximum CO₂ loss that can be obtained with a C6 yeast in this setup is about 450 g CO2 loss (per 40 g/L fermented hexose sugar, which corresponds to a maximum of 563 mg per 50g/liter fermented hexose sugar). By adding in xylose and using a C5 yeast, the setpoint can, also according to Fig. 4, be set above that limit, and thus favor a C5 yeast over a C6 yeast, as a C5 yeast which simultaneous is fermenting xylose and hexose will have a rate advantage over a C6 yeast at low hexose concentrations (e.g. hexose at 5 g/L or less).

Without desiring to be limited to any specific theory, a C5 yeast, e.g. an engineered xylose fermenting S. cerevisiae, express the genes specific for the xylose fermentation ability constitutively, as S. cerevisiae does not recognize xylose as a fermentable sugar and thus is not inducing any fermentation specific genes in the presence of xylose as the only sugar. The xylose fermenting S. cerevisiae strains available today will therefore get a growth rate related benefit from shedding the burden of constitutively expressing the xylose fermentation specific genes, or, in other words, while growing on hexoses, the xylose fermentation ability is inherently disadvantageous from a competitive perspective. This results in yeast populations growing on hexose only to outcompete the desired xylose fermenting yeast populations, e.g. in continues fermentations. Stated differently, the maintenance and expression of xylose fermentation ability in a microorganism is inherently disadvantageous from a competitive perspective in situations where amble glucose and no or little xylose is present. This results in that an introduced yeast population growing on hexose only inevitably will outcompete a xylose fermenting yeast population if glucose is available for growth at all times. Such a subpopulation may thus originate from a single or a few cells in a xylose fermenting population, if one or a few cells accidentally generates an offspring that for example has a gene copying error in the xylose isomerase gene or gene promoter, that prevents the expression of xylose isomerase. The competition advantage of cells having a cancelled the expression of xylose isomerase can however be reversed by maintaining a situation, where the xylose fermentation ability adds to the growth rate.

A continuous fermentation performed according to this invention can be maintained in a state or phase where xylose is present and the hexose/xylose ratio is maintained such that the hexose/xylose fermenting cells has a competitive growth rate advantage over strains that are strictly dependent on hexose fermentation. As shown in the batch fermentation examples above where the sugar mixture includes xylose, and where a C5 yeast has a growth rate advantage, the fermentation is considerably improved if mannose is present at least as part of the hexose sugar, resulting in a shortening of the time needed to complete a batch fermentation, or a stabilization of the xylose fermenting trait in the engineered yeast. Regarding a continuous xylose/glucose fermentation, two advantages of mixing mannose into the fermentation media are thus evident: The xylose fermenting trait is stabilized, allowing a longer fermentation, and the expanded simultaneous fermentation phase allows for setting the setpoint at a later stage of the initial batch fermentation, thus increasing the ethanol concentration in the steady state phase, which will lower the risk of a contamination to build up. Meaning that on average, a longer continuous phase without contamination is possible.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A method of increasing the fermentability of a at least a part of a microbial fermentation process involving co-fermentation of sugars from lignocellulosic biomass to fermentation products, comprising the steps of:
- providing a first lignocellulosic biomass-based stream and a second lignocellulosic biomass-based stream different to the first lignocellulosic biomass-based stream, the first and second lignocellulosic biomass-based streams together comprising xylose, glucose and mannose;
- mixing the first and second lignocellulosic biomass-based streams;
- treating the mixed first and second lignocellulosic biomass-based streams, or treating the first and second lignocellulosic biomass-based streams separately prior to the step of mixing, to provide a fermentation media comprising xylose, glucose and mannose, wherein the mixing of the first and second lignocellulosic biomass-based streams results in a ratio of xylose to mannose in the fermentation media within a target range;
- co-fermenting the fermentation media by means of fermentation microorganisms of yeast to a fermentation product.

2. The method according to claim 1, wherein the target range is different to a ratio of xylose to mannose in the separate first and/or second lignocellulosic biomass-based streams.

3. The method according to any one of the preceding claims, wherein the target range is a predefined target range, the predefined target range being based on an increased fermentation rate during at least a part of the co-fermentation of the fermentation media.

4. The method according to claim 3, wherein the part of the co-fermentation of the fermentation media for which the predefined target range causes an increased fermentation rate comprises the fermentation phase where the utilisation of fermentable sugar changes from primarily glucose to primarily xylose.

5. The method according to any one of the preceding claims, wherein the target range is extending from 0.08 to 13.

6. The method according to any one of the preceding claims, wherein the amount of xylose in the fermentation media is between 5 and 65 wt%, and the amount of mannose in the fermentation media is between 5 and 65 wt%, as compared to the total amount of glucose, mannose and xylose.

7. The method according to any one of the preceding claims, wherein the step of co-fermenting is performed in a continuous, or semi-continuous, manner in a fermentation vessel.

8. The method according to claim 7, comprising the steps of:
- measuring of a residual sugar indicator parameter RSI, which parameter directly or indirectly indicates the concentration of residual sugars, during fermentation in the fermentation vessel;
- determining an RSI setpoint based on a rate of change of the measured residual sugar indicator parameter, such that the RSI setpoint corresponds to a maximum rate of change; and
- automatically adapting the ratio of xylose to mannose in a predetermined manner in response to the measured residual sugar indicator parameter RSI and the RSI setpoint, so as to achieve and maintain the RSI setpoint, whereby efficient co-fermentation of sugars to fermentation products is obtained.

9. The method according to any one of the preceding claims, comprising the step of intentionally increasing the ethanol concentration of the fermentation media, and performing the step of co-fermenting the fermentation media in the presence of an intentionally increased ethanol concentration.

10. The method according to claim 9, further comprising the steps of:
- determining the ethanol concentration in the medium during the step of co-fermenting,
- in response of determining that the concentration of ethanol is below a threshold limit, adjusting the ethanol concentration to be above the threshold limit.

11. The method according to any one of the preceding claims, wherein the yeast is a xylose - engineered yeast.

12. The method according to any one of the preceding claims, wherein one of the first and second lignocellulosic biomass-based streams comprises at least glucose and xylose and are derived from e.g. hardwood, and the other one of the first and second lignocellulosic biomass-based streams comprises at least mannose and are derived from e.g. softwood, such as spruce or pine.

13. Use of mannose to increase the fermentation rate of a microbial fermentation process involving co-fermentation of a fermentation media comprising at least glucose and xylose, wherein the amount of mannose is adapted to reach a ratio of xylose to mannose of the fermentation media within a target range

14. The use of mannose according to claim 13, wherein the target range is based on an increased fermentation rate during at least a part of the co-fermentation of the fermentation media.

15. A system for increasing the fermentability of a microbial fermentation process involving co-fermentation of sugars from lignocellulosic biomass to fermentation products, the system comprising:
- a fermentation unit configured to ferment a fermentation media comprising at least glucose, xylose and mannose;
- means of controlling that a ratio of xylose to mannose of the fermentation media is within a target range.
